# EUROPEAN PATENT APPLICATION

(11) **EP 4 847 486 A1**
(43) Date of publication of application: **02.12.2026**
(21) Application number: 26178850.9
(22) Date of filing: 15.05.2026
(51) Int. Cl.: C07C 227/16, C07C 227/18, C07C 227/40, C07C 229/48

(54) **AN IMPROVED PROCESS FOR PREPARATION AND ISOLATION OF TRANS-4-AMINOCYCLOHEXANE-1-CARBOXYLATE ALKYL ESTER OR SALT THEREOF**

(30) Priority: 16.05.2025 IN 202521047469
(71) Applicant: Aarti Pharmalabs Limited, Vapi, Gujarat 396195 (IN)
(72) Inventor: SALVI, Narendra Jagannath, 421 204 Mumbai (IN); VALDA, Balraju, 400 706 Mumbai (IN); KUMAR, Lalit, 400 706 Mumbai (IN); KAMBLE, Kiran Mohan, 400 706 Mumbai (IN)
(74) Representative: Gassner, Birgitta

(57) **Abstract**

The present invention relates to a process for preparation and separation isolation of *trans-*isomer of 4-aminocyclohexane-1-carboxylate alkyl ester of Formula I or salt thereof with purity ≥ 99.5%, the process comprising: 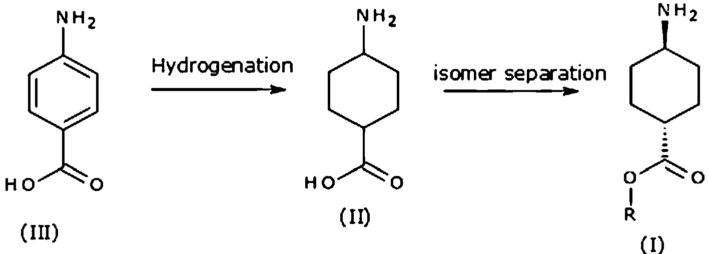 i) hydrogenating 4-aminobenzoic acid of Formula III in presence of a suitable metal catalyst to form 4-aminocyclohexane carboxylic acid of Formula II;
ii) reacting the compound of Formula II with an acidified (1-5)C alcohol;
iii) heating at a temperature in a range of 40°C to 80°C;
iv) cooling the reaction mass;
v) filtering and washing with a (1-5)C alcohol;
vi) distilling the filtrate under vacuum;
vii) adding acetonitrile and a (1-5)C alcohol;
viii) heating at a temperature in a range of 40°C to 80°C;
ix) cooling and isolating pure *trans*-isomer of compound of Formula I or salt thereof,
wherein R is selected from methyl, ethyl, isopropyl, or butyl.

The invention further provides a process for isolation of *trans*-isomer of compound of Formula II by treating a mixture comprising *cis* and *trans*-isomers of compound of Formula II with methanol and isopropanol.

## Description

### Field of the invention

The present invention relates to an industrially scalable process for preparation and isolation of *trans-isomer* of 4-aminocyclohexane-1-carboxylate alkyl ester or salt thereof with purity ≥ 99.5%.

### Background of the invention

*trans*-4-Aminocyclohexane-1-carboxylate alkyl ester or salt thereof of Formula I is an important key intermediate in the manufacturing of optically active compounds such as Glimeperide, Rivumenib and the like.

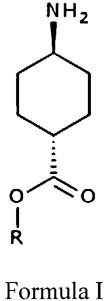

4-Aminocyclohexane-1-carboxylic acid or its (1-4)C alkyl ester being disubstituted ring, exhibit geometric isomerism and have certain arrangements of atoms within molecule. The disubstituted cyclohexane exists as four stereoisomers, two cis-isomers and *two-trans* isomers. Thus, substitutions differ in the relative spatial orientation of the atoms.

Thus, the words *cis-* and *trans-* relate to the relative stereochemistry of amino group and carboxylic acid/ester groups on cyclohexane ring.

In the cis-configuration, both the amino and carboxylic acid/ester groups are on the same plane/face in the most stable chair form.

In the trans-configuration, both the amino and carboxylic acid/ester groups are on the different plane/face in the most stable chair form.

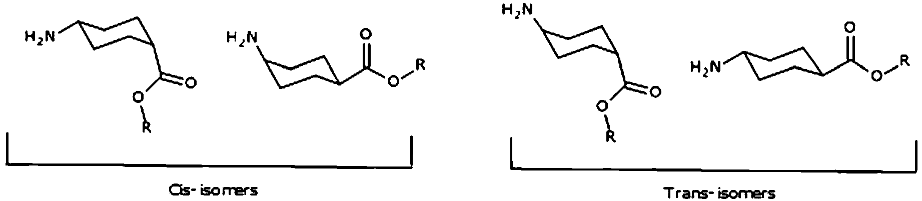

Being in different face/plane, the substitutions are away from each other and due to lesser steric hindrance trans-isomers are more stable. However, isolation of *trans-isomer* from the mixture of *cis* and trans-isomers with the purity equal to or greater than 99.5% is the great challenge.

The prior art references disclose various processes for the preparation and isolation of *trans-*isomer of 4-aminocyclohexane-1-carboxylate alkyl ester, however, none of them assures purity greater than 99%.

FR 1473246 generally claims a process for the preparation of *cis* or *trans* 2-, 3-, or 4-aminocyclohexane carboxylic acids, by hydrogenating the corresponding nitrobenzoates in aqueous solution and in ammonia under pressure, in the presence of a catalyst for hydrogenating the aromatic ring and then evaporating the hydrogenated ammoniacal solution. However, the disclosed example on Kg scale shows the formation of *cis*-isomer assaying 90%. The reaction was carried out at around 102 Kg/cm² (100 bar). Thus, there is no report of specific reaction conditions for isolation of pure *trans*-isomer.

EP3411355B1 claims a one pot process for the preparation of a 4-aminocyclohexane carboxylic acid with a *trans* ratio of more than 75%, comprising reacting 4-aminobenzoic acid using 5% Ruthenium on carbon as catalyst and water as a solvent under basic conditions. The reaction temperature was 100°C and under the hydrogen pressure of 15 bar. After completion of reaction the *cis:trans-*ratio is 1:4.6. The *cis:trans* mixture of 4-aminocyclohexane carboxylic acid obtained was treated with BOC anhydride to form 4-(*tert-*butoxycarbonylamino)cyclohexanecarboxylic acid in *cis:trans* ratio of 1:3.6. The BOC-amino acid thus obtained was treated with potassium carbonate in acetone. Methyl bromide was added under stirring. *Trans*-4-{[(*tert-butoxy*)carbonyl]amino}cyclohexane-1*-*carboxylic acid was precipitated from the *cis*-derivative with 99.1% purity and 62% yield from organic layer. The process uses protection of amine for separation of *trans-*isomer*.*

Accordingly, there is a need for a simple, efficient and industrially feasible process for preparation andisolation of *trans-*isomer of 4-aminocyclohexane-1-carboxylate alkyl ester or salt thereof with purity ≥ 99.5%.

### Objects of the invention

An object of the present invention is to provide an improved process for preparation of *trans-*4-aminocyclohexane-1-carboxylate alkyl ester or salt thereof that is suitable for industrial production.

Another object of the present invention is to provide an improved process for preparation of trans-4-aminocyclohexane-1-carboxylate alkyl ester or salt thereof having higher yield and better purity.

Another object of the present invention is to provide an improved process for preparation of *trans*-4-aminocyclohexane-1-carboxylate alkyl ester or salt thereof which avoids protection and de-protection of amino group.

Another object of the present invention is to provide a process for isolation of *trans-*isomer of 4-aminocyclohexane carboxylic acid from a mixture of *cis* and *trans*-isomers of 4-aminocyclohexane carboxylic acid.

Further, an object of the present invention is to provide a process for preparation and isolation of *trans-*isomer of 4-aminocyclohexane-1-carboxylate alkyl ester or salt thereof with higher purity.

### Summary of the invention

Accordingly, the present invention relates to a process for preparation and isolation of *trans-*isomer of 4-aminocyclohexane-1-carboxylate alkyl ester of Formula I or salt thereof with purity ≥ 99.5%, the process comprising:
i) hydrogenating 4-aminobenzoic acid of Formula III in presence of a metal catalyst to form 4-aminocyclohexane carboxylic acid of Formula II;
ii) reacting the compound of Formula II with an acidified (1-5)C alcohol;
iii) heating the mixture of step (ii);
iv) cooling the reaction mass;
v) filtering and washing with a (1-5)C alcohol;
vi) distilling the filtrate under vacuum;
vii) adding acetonitrile and a (1-5)C alcohol;
viii) heating the mixture of step (vii);
ix) cooling and isolating pure *trans-*isomer of compound of Formula I or salt thereof; wherein R is selected from methyl, ethyl, isopropyl, or butyl.

Further, the present invention relates to a process for isolation of *trans-*isomer of 4-aminocyclohexane carboxylic acid of Formula II, the process comprising treating a mixture comprising *cis* and *trans*-isomers of compound of Formula II, with methanol and isopropanol.

Furthermore, the present invention relates to a process for preparation of *trans-*isomer of 4-aminocyclohexane-1-carboxylate alkyl ester of Formula I or salt thereof, the process comprising reacting *trans-*isomer of 4-aminocyclohexane carboxylic acid of Formula II with an acidified (1-5)C alcohol.

Still further, the present invention relates to a process for isolation of *trans-*isomer of -aminocyclohexane-1-carboxylate alkyl ester of Formula I or salt thereof, the process comprising treating a residue containing *trans-*isomer of 4-aminocyclohexane-1-carboxylate alkyl ester of Formula I or salt thereof, with acetonitrile and a (1-5)C alcohol selected from methanol, ethanol, isopropanol, isobutanol, butanol, or pentanol.

The present invention further relates to a process for preparation and isolation of *trans-*isomer of 4-aminocyclohexane-1-carboxylate alkyl ester of Formula I or salt thereof, telescopically.

### Detailed description of the invention

The foregoing objects of the present invention are accomplished and the problems and shortcomings associated with the prior art, techniques and approaches are overcome by the present invention as described below in the preferred embodiments.

In an aspect, the present invention relates to a process for preparation and isolation of *trans-*isomer of 4-aminocyclohexane-1-carboxylate alkyl ester of Formula I or salt thereof with purity ≥ 99.5%, wherein R is selected from (1-4)C alkyl. The process comprises:
i) hydrogenating 4-aminobenzoic acid of Formula III in presence of a metal catalyst to form 4-aminocyclohexane carboxylic acid of Formula II; 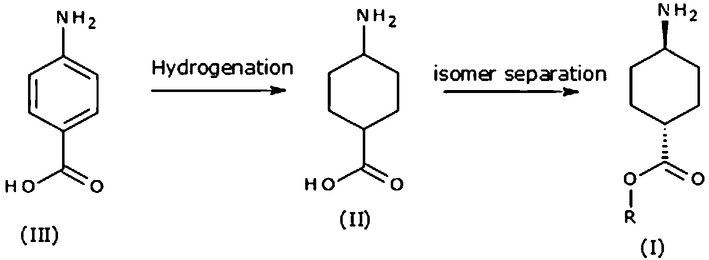
ii) reacting the compound of Formula II with an acidified (1-5)C alcohol;
iii)heating the mixture of step (ii);
iv) ooling the reaction mass;
v) filtering and washing with a (1-5)C alcohol;
vi)distilling the filtrate under vacuum;
vii) adding acetonitrile and a (1-5)C alcohol;
viii) heating the mixture of step (vii);
ix)cooling and isolating pure *trans-*isomer of compound of Formula I or salt thereof, wherein R is selected from methyl, ethyl, isopropyl, or butyl.

The metal catalyst used in stage (i) is selected from ruthenium on carbon (Ru/C), rhodium on carbon, platinum on carbon, palladium on carbon, or Raney Ni, preferably the metal catalyst is ruthenium on carbon.

The reaction in stage (i) is carried out substantially in aqueous medium.

The hydrogenation reaction in stage (i) is carried out at 90°C to 150°C, preferably at 100°C to 140°C, particularly at 120°C to 130°C. The reaction proceeds under 30-40 kg/cm² of hydrogen pressure.

Preferably, the reaction is carried out in a suitable alkali selected from alkali metal hydroxide or alkali metal alkoxide. The alkali metal hydroxide is selected from sodium hydroxide, potassium hydroxide, lithium hydroxide or caesium hydroxide. The alkali metal alkoxide is selected from sodium methoxide, sodium ethoxide, sodium isopropoxide, sodium butoxide and the like. The reaction is preferably carried out in presence of potassium hydroxide.

Ru/C is recycled and reused in the next batch along with top-up loading of fresh Ru/C. The reuse of the catalyst makes the process economical.

In an embodiment, after stage (i), the compound of Formula II is extracted from the reaction mass with methanol, preferably, by heating the reaction mass with methanol at 60°C to 65°C for 2 hours. Subsequent to heating, the mixture is filtered, and the filtrate is used in stage (ii) of the process.

The acidified (1-5)C alcohol used in stage (ii) comprises a (1-5)C alcohol selected from methanol, ethanol, isopropanol, isobutanol, butanol, or pentanol, and an acid selected from hydrochloric acid or sulfuric acid. Preferably, the (1-5)C alcohol is methanol and acid is hydrochloric acid.

Preferably, the pH of the reaction mixture in stage (ii) is adjusted to be 1.

Heating in stage (iii) is carried out at 40°C to 80°C, preferably at 45°C to 75°C, particularly at 50°C to 70°C, for 7-15 hours.

After completion of reaction, the mixture in stage (iv) is cooled to 5°C to 10°C.

The (1-5)C alcohol used in stage (v) is selected from methanol, ethanol, isopropanol, isobutanol, butanol, or pentanol, preferably the (1-5)C alcohol is isopropanol.

Distillation of the filtrate in stage (vi) is carried out under vacuum below 60°C till a semi-solid is obtained.

(1-5C) alcohol and acetonitrile added in stage (vii) is in a ratio of 30:70-5:95, preferably in the ratio of 20:85, particularly in the ratio of 10:90.

The (1-5)C alcohol used in stage (vii) is selected from methanol, ethanol, isopropanol, isobutanol, butanol, or pentanol, preferably the(1-5)C alcohol is methanol.

Heating in stage (viii) is carried out at 50°C to 70°C, preferably at 60°C to 65°C, for 1 to 2 hours.

After completion of the reaction in stage (viii), the mass obtained is cooled at 30°C to 40°C in stage (ix) and stirred for 1 to 2 hours.

The *trans-*isomer obtained in stage (ix) is isolated by filtration with ≥ 99.5% purity.

The process of the present invention is a telescopic process.

The use of an acidified alcohol in the reaction of stage (ii), results in a higher yield of the product, *trans*-4-aminocyclohexane-1-carboxylate alkyl ester of Formula I or salt thereof. The acidified alcohol not only aids in the esterification of the compound of Formula II but also acts as a source of acid for the formation of the salt of the ester, and further aids in the purification of the *trans-*isomer of compound of Formula I or its salts. Using acetonitrile and (1-5)C alcohol, in stage (vii), avoids the steps of protection and deprotection of amino groups, which are usually performed in the isolation of the *trans-*isomer of the compound of Formula I or its salts. Thereby, the process provides a high yield of highly pure *trans-*isomer of the compound of Formula I or salts thereof, while being simple, economical, and industrially feasible.

In another aspect, the present invention relates to a process for isolation of *trans-*isomer of 4-aminocyclohexane carboxylic acid of Formula II, the process comprising treating a mixture comprising *cis* and *trans*-isomers of compound of Formula II, with methanol and isopropanol (IPA).

The 4-aminocyclohexane carboxylic acid of Formula II is the product of hydrogenation of 4-aminobenzoic acid of Formula III, which has been described above.

In an embodiment, after the hydrogenation reaction, the reaction mass is cooled to a temperature in a range of 30°C to 35°C. Then, the reaction mass is filtered. Following filtration, pH of the filtrate is adjusted in a range of 4 to 4.5. Preferably, pH adjustment is done with a mineral acid such as concentrated hydrochloric acid. After pH adjustment, water is completely distilled off from the filtrate to obtain a mixture of comprising *cis* and *trans*-isomers of compound of Formula II. Preferably, the distillation is performed under vacuum at a temperature below 65°C.

The treatment of mixture comprising *cis* and *trans*-isomers of compound of Formula II involves adding methanol to the mixture. The resulting mass is then heated to a temperature in the range of 60°C to 65°C. Preferably, the resulting mass is stirred for 2 hours while maintaining the temperature in the specified range. The mass is then filtered to separate a solid and a filtrate. Subsequently, the filtrate is distilled under vacuum at a temperature below 60°C to obtain a semi-solid mass. The semi-solid mass is cooled to a temperature in the range of 30°C to 35°C, and isopropanol is added. Following the addition of isopropanol, the mixture is heated to a temperature in the range of 60°C to 65°C. Preferably, the mixture is stirred for 2 hours while maintaining the temperature in the specified range. The mixture is then cooled to a temperature in the range of 30°C to 35°C. The *trans-*isomer of 4-aminocyclohexane carboxylic acid of Formula II is isolated from the mixture as a solid by filtration. The solid obtained after filtration is washed with isopropanol and dried under vacuum at a temperature in the range of 60°C to 70°C. Preferably, the drying is carried out for a duration of 10 to 11 hours.

The use of methanol and isopropanol for isolation provides a higher yield of *trans-*isomer of 4-aminocyclohexane carboxylic acid of Formula II.

In another aspect, the present invention relates to a process for preparation of *trans-*isomer of 4-aminocyclohexane-1-carboxylate alkyl ester of Formula I or salt thereof from the *trans-*isomer of 4-aminocyclohexane carboxylic acid of Formula II. The process comprises reacting the *trans-*isomer of Formula II, prepared by the process described above, with an acidified (1-5)C alcohol.

The process steps, the process parameters, and the acidified (1-5)C alcohol have been described above.

In another aspect, the present invention relates to a process for isolation of *trans-*isomer of 4-aminocyclohexane-1-carboxylate alkyl ester of Formula I or salt thereof. The process comprises treating a residue containing *trans-*isomer of 4-aminocyclohexane-1-carboxylate alkyl ester of Formula I or salt thereof, prepared by the the process described above, with acetonitrile and a (1-5)C alcohol selected from methanol, ethanol, isopropanol, isobutanol, butanol, or pentanol.

The process steps and the process parameters have been described above.

In particular, the invention is further described by one or more of the following items.
1. A process for preparation and isolation of *trans-isomer* of 4-aminocyclohexane-1-carboxylate alkyl ester of Formula I or salt thereof with purity ≥ 99.5%, 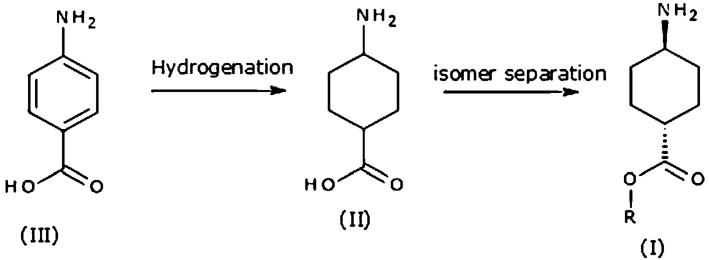 the process comprising:
   i) hydrogenating 4-aminobenzoic acid of Formula III in presence of a metal catalyst to form 4-aminocyclohexane carboxylic acid of Formula II;
   ii) reacting the compound of Formula II with an acidified (1-5)C alcohol;
   iii) heating the mixture of step (ii);
   iv) cooling the reaction mass;
   v) filtering and washing with a (1-5)C alcohol;
   vi) distilling the filtrate under vacuum;
   vii) adding acetonitrile and a (1-5)C alcohol;
   viii) heating the mixture of step (vii);
   ix) cooling and isolating pure *trans-*isomer of compound of Formula I or salt thereof, wherein R is selected from methyl, ethyl, isopropyl, or butyl.
2. The process of item 1, wherein the metal catalyst used in stage (i) is selected from ruthenium on carbon, rhodium on carbon, platinum on carbon, palladium on carbon or Raney Ni.
3. The process of item 1 or 2, wherein the metal catalyst used in stage (i) is ruthenium on carbon.
4. The process of any one of items 1 to 3, wherein the metal catalyst is recovered after completion of stage (i) and reused in stage (i) of a subsequent batch of the process.
5. The process of item 1, wherein reaction in stage (i) is carried out substantially in aqueous medium.
6. The process of item 1, wherein the hydrogenation reaction in stage (i) is carried out at 90°C to 150°C, preferably at 100°C to 140°C.
7. The process of item 1 or 6, wherein the hydrogenation reaction in stage (i) is carried out at 120°C to 130°C.
8. The process of item 1, wherein the hydrogenation reaction in stage (i) is carried out under 30-40 kg/cm² of hydrogen pressure.
9. The process of item 1, wherein the hydrogenation reaction in stage (i) is carried out in presence of an alkali selected from alkali metal hydroxide or alkali metal alkoxide.
10. The process of item 9, wherein, the alkali metal hydroxide is selected from sodium hydroxide, potassium hydroxide, lithium hydroxide, or caesium hydroxide, and the alkali metal alkoxide is selected from sodium methoxide, sodium ethoxide, sodium isopropoxide, or sodium butoxide.
11. The process of item 1, 9, or 10, wherein the hydrogenation reaction in stage (i) is preferably carried out in presence of potassium hydroxide.
12. The process of item 1, wherein the acidified (1-5)C alcohol used in stage (ii) comprises a (1-5)C alcohol selected from methanol, ethanol, isopropanol, isobutanol, butanol, or pentanol, and an acid selected from hydrochloric acid or sulfuric acid.
13. The process of item 1 or 12, wherein the acidified (1-5)C alcohol used in stage (ii) comprises methanol and hydrochloric acid.
14. The process of item 1, wherein the heating in stage (iii) is carried out at 40°C to 80°C, preferably at 45°C to 75°C.
15. The process of item 1 or 14, wherein the heating in stage (iii) is carried out at 50°C to 70°C.
16. The process of item 1 or 15, wherein the reaction mass in stage (iv) is cooled to 5°C to 10°C.
17. The process of item 1, wherein the (1-5)C alcohol used in stage (v) is selected from methanol, ethanol, isopropanol, isobutanol, butanol, or pentanol.
18. The process of item 1 or 17, wherein the (1-5)C alcohol used in stage (v) is isopropanol.
19. The process of item 1, wherein distillation of the filtrate in stage (vi) carried out under vacuum below 60°C till a semi-solid is obtained.
20. The process of item 1, wherein the (1-5)C alcohol used in stage (vii) is selected from methanol, ethanol, isopropanol, isobutanol, butanol, or pentanol.
21. The process of item 1 or 20, wherein the (1-5)C alcohol used in stage (vii) is methanol.
22. The process of item 1, wherein (1-5)C alcohol and acetonitrile added in stage (vii) is in a ratio of 30:70-5:95.
23. The process of item 1 or 22, wherein (1-5)C alcohol and acetonitrile added in stage (vii) is in the ratio of 20:85, particularly in the ratio of 10:90.
24. The process of item 1, 22, or 23, wherein (1-5)C alcohol and acetonitrile added in stage (vii) is in the ratio of 10:90.
25. The process of item 1, wherein heating in stage (viii) is carried out at 50°C to 70°C.
26. The process of item 1, wherein the mass obtained in stage (viii) is cooled at 30°C to 40°C in stage (ix).
27. The process of item 1, wherein *trans-*isomer obtained in stage (ix) is isolated by filtration.
28. The process of any one of items 1 to 27, wherein the process is a telescopic process.
29. A process for isolation of *trans-*isomer of 4-aminocyclohexane carboxylic acid of Formula II, 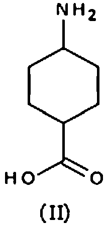 the process comprising treating a mixture comprising *cis* and *trans*-isomers of compound of Formula II with methanol and isopropanol.
30. The process of item 29, wherein the process comprises adding methanol to the mixture comprising *cis* and *trans*-isomers of compound of Formula II at 35°C, 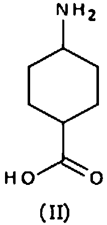 heating the mixture to a temperature of 60°C to 65°C, filtering and distilling the filtrate under vacuum at a temperature below 60°C to obtain a semi-solid residue, cooling the semi-solid residue to a temperature of 30°C to 35°C, adding isopropanol to the semisolid residue, and heating to a temperature of 60°C to 65°C to obtain *trans-*isomer of compound of Formula II.
31. A process for preparation of *trans-*isomer of 4-aminocyclohexane-1-carboxylate alkyl ester of Formula I or salt thereof, 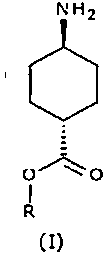 the process comprising reacting *trans*-isomer of 4-aminocyclohexane carboxylic acid of Formula II with an acidified (1-5)C alcohol.
32. The process of item 31, wherein the process comprises heating the reaction mass comprising *trans-*isomer of 4-aminocyclohexane carboxylic acid of Formula II 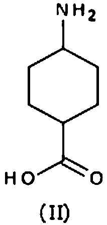 and acidified (1-5)C alcohol at 40°C to 80°C; cooling the reaction mass at 5°C to 10°C; filtering and washing with (1-5)C alcohol selected from methanol, ethanol, isopropanol, isobutanol, butanol, or pentanol; and distilling the filtrate under vacuum at a temperature below 60°C.
33. The process of item 31 or 32, wherein the acidified (1-5)C alcohol comprises a (1-5)C alcohol selected from methanol, ethanol, isopropanol, isobutanol, butanol, or pentanol, and an acid selected from hydrochloric acid or sulfuric acid.
34. A process for isolation of *trans-*isomer of 4-aminocyclohexane-1-carboxylate alkyl ester of Formula I or salt thereof, 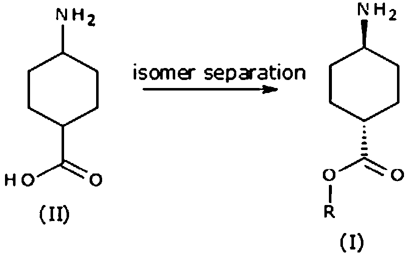 the process comprising treating a residue containing *trans-*isomer of 4-aminocyclohexane-1-carboxylate alkyl ester of Formula I or salt thereof with acetonitrile and an (1-5)C alcohol selected from methanol, ethanol, isopropanol, isobutanol, butanol, or pentanol.
35. The process of item 34, wherein the process further comprises heating the mass comprising *trans-*isomer of 4-aminocyclohexane-1-carboxylate alkyl ester of Formula I or salt thereof, 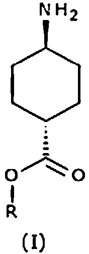
   acetonitrile, and the (1-5)C alcohol at 50°C to 70°C;
   cooling the mass at 30°C to 40°C; and
   isolating pure *trans-*isomer of compound of Formula I or salt thereof, wherein R is selected from methyl, ethyl, isopropyl, or butyl.

### EXAMPLES

Only a few examples and implementations are disclosed. Variations, modifications, and enhancements to the described examples and implementations and other implementations can be made based on what is disclosed.

Examples are set forth herein below and are illustrative of different amounts and types of reactants and reaction conditions that can be utilized in practicing the disclosure. It will be apparent, however, that the disclosure can be practiced with other amounts and types of reactants and reaction conditions than those used in the examples, and the resulting devices have various different properties and uses in accordance with the disclosure above and as pointed out hereinafter.

### Example 1

### Preparation of 4-aminocyclohexane carboxylic acid

Potassium hydroxide (53.2 g) was added to the water (600 ml) and stirred for 10 to 15 minutes at 30-35°C. 4-Aminobenzoic acid (100 g) of Formula III was charged to the potassium hydroxide clear solution obtained and stirred for 10 to 15 minutes. Activated charcoal (5 g) was added and the mixture was stirred for 30 minutes at 30°C to 35°C.

The mixture was filtered through hyflow and 5% Ru/C (6 g) was charged. Nitrogen pressure of 2 kg/cm² was applied and released. Hydrogen pressure of 2 kg/cm² was applied and released. The temperature of the reaction mixture was raised to 120°C to 125°C. Hydrogen pressure of 35 kg/cm² was applied and maintained for 10 to 11 hours.

After the reaction completion, the mass was cooled to 30°C to 35°C and the mass was filtered through hyflow bed. The pH of the filtrate was adjusted between 4 to 4.5 using conc. HCl. Water was completely distilled off under vacuum below 65°C. The mass obtained contained a mixture of cis-isomer and *trans-isomer* of 4-aminocyclohexane carboxylic acid. This was used for further isolation of *trans-isomer.*

The catalyst Ru/C was recovered and reused in the next batch along with fresh top up catalyst.

Below table shows the consumption recovered catalyst along with a quantity of fresh catalyst.

| **Cycle** | **Ru/C Loading (w/w)** | **Conversion** |
|---|---|---|
| R1 | 6% fresh | 48% |
| R2 | Recycled from R1+3% top up | 47.5% |
| R3 | Recycled from R2+1.5% top up | 47% |
| R4 | Recycled from R3+0.75% top up | 48% |
| R5 | Recycled from R4+0.375% top up | 47% |

Thus, the recovered catalyst along with a quantity of fresh catalyst efficiently hydrogenates the 4-aminobenzoic acid to 4-aminocyclohexane carboxylic acid with approximately the same yield as that of obtained with fresh catalyst.

### Example 2

### Preparation of methyl trans-4-aminocyclohexane carboxylic acid hydrochloride

4-Aminocyclohexane carboxylic acid (100 g) of Formula II, prepared in Example 1 was charged to methanol (1000 mL) at 30°C to 35°C and stirred for 10 to 15 minutes. pH of the solution was adjusted to 1 using 3M methanolic HCl. The reaction mixture was heated at 60°C to 65°C and the temperature was maintained for 10 to 12 hours. The mass was cooled gradually to 5°C to 10°C. The mass was filtered and washed with methanol (50 mL). Filtrate was distilled under vacuum below 60°C till the semi-solid residue was obtained.

Acetonitrile (900 mL) was charged followed by addition of methanol (100 mL). The mass was heated at 60°C to 65°C and further stirred for 1 hour. The mass was gradually cooled to 30°C to 35°C and stirred for 1 hour. The solid was filtered and washed with the mixture of acetonitrile and methanol (150 mL : 25 mL). The wet solid obtained was suck dried and further dried under vacuum at 60°C to 70°C for 10 to 12 hours.
Yield = 70 g (51.7%)
HPLC Purity:
   *trans-isomer* - 99.5%
   cis-isomer - ND

### Example 3

### Isolation of trans-4-aminocyclohexane carboxylic acid

4-aminocyclohexane carboxylic acid was prepared as described in Example 1. The product contained a mixture of *cis* and *trans*-isomers of 4-aminocyclohexane carboxylic acid. *trans*-4-aminocyclohexane carboxylic acid was isolated from the mixture by treating it with methanol and isopropanol as described below:
Methanol (500 mL) was added to the mixture of *cis* and *trans*-isomers of 4- aminocyclohexane carboxylic acid obtained in Example 1. The mixture was heated to 60°C to 65°C and stirred for 2 hours. After heating, the mixture was filtered to separate a solid and a filtrate. The filtrate was distilled under vacuum below 60°C till a semi-solid residue was obtained. The residue was cooled to 30°C to 35°C and isopropanol (IPA) (500 mL) was added. The mixture was heated to 60°C to 65°C and stirred for 2 hours. After heating, the mixture was cooled to 30°C to 35°C and stirred for 1 hour. The solid was filtered off and washed with IPA (100 mL). The wet solid was suck dried and further dried under vacuum at 60°C to 70°C for 10 to 11 hours to obtain *trans*-4-aminocyclohexane carboxylic acid.

### Example 4

### Preparation of methyl trans-4-aminocyclohexane carboxylic acid hydrochloride

*Trans*-4-aminocyclohexane carboxylic acid of Example 3 was heated with 3M methanolic HCl at 60°C to 65°C for 10 to 12 hours. Following heating, the reaction mass was cooled gradually to 5°C to 10°C. The mass was filtered and washed with methanol (50 mL). Filtrate was distilled under vacuum below 60°C till a semi-solid residue containing methyl trans-4-aminocyclohexane carboxylic acid hydrochloride was obtained.

### Example 5

### Isolation of methyl trans-4-aminocyclohexane carboxylic acid hydrochloride

Acetonitrile (900 mL) and methanol (100 mL) were added to the residue prepared in Example 4, and the mixture was heated at 60°C to 65°C and stirred for 1 hour. The mass was gradually cooled to 30°C to 35°C and stirred for 1 hour. The solid was filtered and washed with the mixture of acetonitrile and methanol (150 mL: 25 mL). The wet solid obtained was suck dried and further dried under vacuum at 60°C to 70°C for 10 to 12 hours.
HPLC Purity:
*trans-isomer* - 99.5%
cis-isomer - ND

### Example 6

### Telescopic synthesis of methyl trans-4-aminocyclohexane carboxylic acid hydrochloride

Potassium hydroxide (53.2 g) was added to the water (600 mL) and stirred for 10 to 15 minutes at 30°C to 35°C. 4-Aminobenzoic acid (100 g) of Formula III was charged to the potassium hydroxide clear solution obtained and stirred for 10 to 15 minutes. Activated charcoal (5 g) was added and the mixture was stirred for 30 minutes at 30°C to 35°C.

The mixture was filtered through hyflow and 5% Ru/C (6 g) was charged. Nitrogen pressure of 2 kg/cm² was applied and released. Hydrogen pressure of 2 kg/cm² was applied and released. The temperature of the reaction mixture was raised to 120°C to 125°C. Hydrogen pressure of 35 kg/cm² was applied and maintained for 10 to 11 hours.

After the reaction completion the mass was cooled to 30°C to 35°C and the mass was filtered through hyflow bed. Adjust the pH of the solution between 4 to 4.5 using conc. HCl. Water was completely distilled off under vacuum below 65°C.

The reaction mass was cooled and methanol (500 mL) was added to semi-solid at 35°C. The temperature was raised to 60°C to 65°C and stirred for 2 hours to extract the compound Formula II in methanol. The mixture was filtered and suck dried. pH of the filtrate was adjusted to 1 using 3M methanolic HCl. Then, the reaction mixture was heated at 60°C to 65°C and the temperature was maintained for 10 to 12 hours. The mass was cooled gradually to 5°C to 10°C. The mass was filtered and washed with methanol (50 mL). Filtrate was distilled under vacuum below 60°C till the semi-solid residue was obtained.

Acetonitrile (900 mL) was charged followed by addition of methanol (100 mL). The mass was heated at 60°C to 65°C and further stirred for 1 hour. The mass was gradually cooled to 30°C to 35°C and stirred for 1 hour. The solid was filtered and washed with the mixture of acetonitrile and methanol (150 mL: 25 mL). The wet solid obtained was suck dried and further dried under vacuum at 60°C to 70°C for 10 to 12 hours.
Yield = 45 g (31.9%)
HPLC purity:
   *trans-*isomer = 99.5%
   *cis*-isomer = ND

## Claims

1. A process for preparation and isolation of *trans-*isomer of 4-aminocyclohexane-1-carboxylate alkyl ester of Formula I or salt thereof with purity ≥ 99.5%, 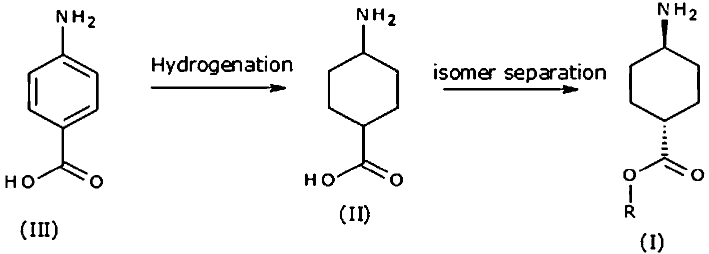 the process comprising:
i) hydrogenating 4-aminobenzoic acid of Formula III in presence of a metal catalyst to form 4-aminocyclohexane carboxylic acid of Formula II;
ii) reacting the compound of Formula II with an acidified (1-5)C alcohol;
iii) heating the mixture of step (ii);
iv) cooling the reaction mass;
v) filtering and washing with a (1-5)C alcohol;
vi) distilling the filtrate under vacuum;
vii) adding acetonitrile and a (1-5)C alcohol;
viii) heating the mixture of step (vii);
ix) cooling and isolating pure *trans*-isomer of compound of Formula I or salt thereof, wherein R is selected from methyl, ethyl, isopropyl, or butyl.

2. The process as claimed in claim 1, wherein,
the reaction in stage (i) is carried out substantially in aqueous medium, and/or the metal catalyst used in stage (i) is selected from ruthenium on carbon, rhodium on carbon, platinum on carbon, palladium on carbon or Raney Ni, and/or the metal catalyst used in stage (i) is ruthenium on carbon, and/or the hydrogenation reaction in stage (i) is carried out under 30-40 Kg/cm² of hydrogen pressure, and/or
the hydrogenation reaction in stage (i) is carried out at 90°C to 150°C, preferably at 100°C to 140°C, and/or
the metal catalyst is recovered after completion of stage (i) and reused in stage (i) of a subsequent batch of the process.

3. The process as claimed in claim 1 or 2, wherein the hydrogenation reaction in stage (i) is carried out at 120°C to 130°C.

4. The process as claimed in claim 1 or 2, wherein the hydrogenation reaction in stage (i) is carried out in presence of an alkali selected from alkali metal hydroxide or alkali metal alkoxide,
wherein, the alkali metal hydroxide is selected from sodium hydroxide, potassium hydroxide, lithium hydroxide, or caesium hydroxide, and the alkali metal alkoxide is selected from sodium methoxide, sodium ethoxide, sodium isopropoxide, or sodium butoxide.

5. The process as claimed in claim 1 or 4, wherein the hydrogenation reaction in stage (i) is preferably carried out in presence of potassium hydroxide.

6. The process as claimed in claim 1, wherein the acidified (1-5)C alcohol used in stage (ii) comprises a (1-5)C alcohol selected from methanol, ethanol, isopropanol, isobutanol, butanol, or pentanol, and an acid selected from hydrochloric acid or sulfuric acid, and/or the heating in stage (iii) is carried out at 40°C to 80°C, preferably at 45°C to 75°C, and/or the reaction mass in stage (iv) is cooled to 5°C to 10°C, and/or
the (1-5)C alcohol used in stage (v) is selected from methanol, ethanol, isopropanol, isobutanol, butanol, or pentanol, and/or
distillation of the filtrate in stage (vi) carried out under vacuum below 60°C till a semi-solid is obtained, and/or
the (1-5)C alcohol used in stage (vii) is selected from methanol, ethanol, isopropanol, isobutanol, butanol, or pentanol, and/or
(1-5)C alcohol and acetonitrile added in stage (vii) is in a ratio of 30:70-5:95, and/or heating in stage (viii) is carried out at 50°C to 70°C, and/or
the mass obtained in stage (viii) is cooled at 30°C to 40°C in stage (ix), and/or the trans-isomer obtained in stage (ix) is isolated by filtration.

7. The process as claimed in claim 1 or 6, wherein the acidified (1-5)C alcohol used in stage (ii) comprises methanol as the (1-5)C alcohol and hydrochloric acid as the acid.

8. The process as claimed in claim 1 or 6, wherein the heating in stage (iii) is carried out at 50°C to 70°C.

9. The process as claimed in claim 1 or 6, wherein the (1-5)C alcohol used in stage (v) is isopropanol.

10. The process as claimed in claim 1 or 6, wherein the (1-5)C alcohol used in stage (vii) is methanol.

11. The process as claimed in claim 1 or 6, wherein (1-5)C alcohol and acetonitrile added in stage (vii) is in the ratio of 20:85 or in the ratio of 10:90.

12. The process as claimed in any one of claims 1 to 11, wherein the process is a telescopic process.

13. A process for isolation of *trans-*isomer of 4-aminocyclohexane carboxylic acid of Formula II, 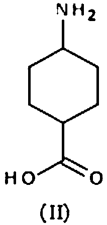 the process comprising treating a mixture comprising *cis* and *trans*-isomers of compound of Formula II with methanol and isopropanol
wherein the treatment comprises,
adding methanol to the mixture comprising *cis* and *trans*-isomers of compound of Formula II at 35°C,
heating the mixture to a temperature of 60°C to 65°C,
filtering and distilling the filtrate under vacuum at a temperature below 60°C to obtain a semi-solid residue,
cooling the semi-solid residue to a temperature of 30°C to 35°C,
adding isopropanol to the semisolid residue, and heating to a temperature of 60°C to 65°C to obtain *trans-*isomer of compound of Formula II.

14. A process for preparation of *trans-*isomer of 4-aminocyclohexane-1-carboxylate alkyl ester of Formula I or salt thereof, 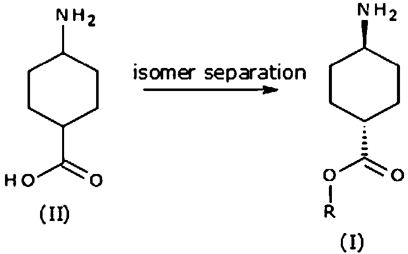 the process comprising reacting *trans-*isomer of 4-aminocyclohexane carboxylic acid of Formula II with an acidified (1-5)C alcohol,
heating the reaction mass comprising *trans-*isomer of 4-aminocyclohexane carboxylic acid of Formula II and the acidified (1-5)C alcohol at 40°C to 80°C;
cooling the reaction mass at 5°C to 10°C;
filtering and washing with (1-5)C alcohol selected from methanol, ethanol, isopropanol, isobutanol, butanol, or pentanol; and
distilling the filtrate under vacuum at a temperature below 60°C,
wherein the acidified (1-5)C alcohol comprises a (1-5)C alcohol selected from methanol, ethanol, isopropanol, isobutanol, butanol, or pentanol, and an acid selected from hydrochloric acid or sulfuric acid.

15. A process for isolation of *trans-*isomer of 4-aminocyclohexane-1-carboxylate alkyl ester of Formula I or salt thereof, 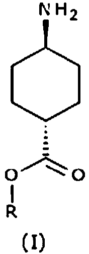 the process comprising treating a residue containing *trans-*isomer of 4-aminocyclohexane-1-carboxylate alkyl ester of Formula I or salt thereof with acetonitrile and an (1-5)C alcohol selected from methanol, ethanol, isopropanol, isobutanol, butanol, or pentanol,
wherein the treatment comprises,
heating the mass comprising *trans-*isomer of 4-aminocyclohexane-1-carboxylate alkyl ester of Formula I or salt thereof, acetonitrile, and the (1-5)C alcohol at 50°C to 70°C;
cooling the mass at 30°C to 40°C; and
isolating pure *trans-*isomer of compound of Formula I or salt thereof,
wherein R is selected from methyl, ethyl, isopropyl, or butyl.
